# EUROPEAN PATENT SPECIFICATION

(11) **EP 2 419 002 B1**
(45) Date of publication and mention of the grant of the patent: **08.07.2015**
(21) Application number: 10714091.5
(22) Date of filing: 08.04.2010
(51) Int. Cl.: A61B 5/00, A61B 5/16

(54) **METHOD AND COMPUTER PROGRAM FOR PROCESSING A PHYSIOLOGICAL SIGNAL**
VERFAHREN UND COMPUTERPROGRAMM ZUR VERARBEITUNG EINES PHYSIOLOGISCHEN SIGNALS
PROCÉDÉ ET PROGRAMME D'ORDINATEUR DE TRAITEMENT D'UN SIGNAL PHYSIOLOGIQUE

(30) Priority: 15.04.2009 EP 09157909
(43) Date of publication of application: 22.02.2012
(73) Proprietor: Koninklijke Philips N.V., 5656 AE Eindhoven (NL)
(72) Inventor: VAN DEN EERENBEEMD, Jacobus, M., A., NL-5656 AE Eindhoven (NL); CROMPVOETS, Floris, M., H., NL-5656 AE Eindhoven (NL); BROKKEN, Dirk, NL-5656 AE Eindhoven (NL); DE VRIES, Jan, J., G., NL-5656 AE Eindhoven (NL); LEMMENS, Paul, M., C., NL-5656 AE Eindhoven (NL)
(74) Representative: Kroeze, Johannes Antonius
(86) International application number: PCT/IB2010/051514
(87) International publication number: WO 2010/119374

(56) References cited:
- EP-A1- 1 071 055
- WO-A2-2006/138164
- WO-A2-2009/016490
- JP-A- H04 175 079
- KR-B1- 100 869 245
- US-A1- 2006 155 398
- KATSUKURA M ET AL: "Life pattern sensor with non-intrusive appliance monitoring" 2009 DIGEST OF TECHNICAL PAPERS INTERNATIONAL CONFERENCE ON CONSUMER ELECTRONICS (ICCE) IEEE PISCATAWAY, NJ, USA, 10 January 2009 (2009-01-10), - 14 January 2009 (2009-01-14) page 2 PP., XP002587475 ISBN: 978-1-4244-2558-7
- CHING-HU LU ET AL: "Hide and not easy to seek: a hybrid weaving strategy for context-aware service provision in a smart home" 2008 IEEE ASIA-PACIFIC SERVICES COMPUTING CONFERENCE (APSCC 2008) IEEE PISCATAWAY, NJ, USA, 2008, pages 595-600, XP002587480 ISBN: 978-0-7695-3473-2

## Description

### FIELD OF THE INVENTION

The invention relates to a method of signal processing, including:
obtaining at least one physiological signal from a system arranged to monitor at least one user.

The invention also relates to a computer program.

### BACKGROUND OF THE INVENTION

US 2008/0200774 relates to a healthcare system comprising a shell configured to be worn around an ear of a subject. At least one physiological sensor is provided to the shell.for measuring and outputting a physiological variable representing a physiological condition of the subject. At least one activity sensor is provided to the shell for measuring and outputting an activity variable representing activity of the subject. At least one environment sensor is provided to the shell for measuring and outputting an environment variable representing the subject's environment. The system comprises a processing module for processing the physiological, activity and environment variables and for generating an output signal based on the physiological, activity and environment variables. The activity sensors can continuously monitor the subject's physical activity in XYZ dimensions for motion detection, including fall detection. In an embodiment, the environment sensor outputs an environmental temperature.

A problem of the known device is that it only uses motion sensors to interpret the physiological data. This is less of a problem where the object of such a device is to monitor a person's physical health. However, when it comes to making inferences about a person's mental state, an interpretation based only on further data characterizing the person's motion can be inadequate.

A behavior detecting apparatus for controlling an appliance is known from EP 1071 055.

### SUMMARY OF THE INVENTION

It is an object of the invention to provide a method, system and computer program of the types mentioned above that enable an automated system more accurately to discern between changes in physiological signal values due to changes in a person's mental state and those due to other influences.

This object is achieved by the method according to claim 1.

The physiological signals need not necessarily be transmitted over a relatively long range. Instead, a body sensor network can relay physiological signals to a signal processing device that is carried by the user or in close vicinity. The first data at least based on second data representative of at least one characteristic of electrical current can be communicated to the portable signal processing device over a longer distance. Further, the invention takes account of the fact that the directionality or strength of wireless signals can be used to advantage to locate a receiver of such signals.

Physiological signals representative of certain physiological phenomena such as heart rate, skin conductance and changes therein are quite highly correlated to a person's mental state (mood or emotional state). Thus, obtaining such signals contributes to the accuracy with which an automated system infers a person's mental state. However, some signal artifacts are due to other influences, for example the type of activity the person is engaging in or has recently engaged in. By obtaining data representative of at least one characteristic of electrical current drawn by at least one appliance in an environment in which the at least one user is present, an inference about the person's activity can be made. An automated system using the data to interpret the at least one physiological signal is therefore capable of identifying changes in mental state more effectively, and to adjust its output accordingly.

This embodiment can be implemented without major adaptation of the electrical appliances in the environment. In particular, the system that processes the physiological signals need not be provided with a communication link to each of the appliances.

In a variant of this embodiment, each monitoring device is comprised in an insert, the insert including a plug for insertion in a wall-mounted socket and at least one socket for receiving a power plug of an electrical appliance.

This variant can be implemented without major adaptation of the environment, in particular alterations to the mains supply in a building. A further effect is that inferences can be made as to the location or locations of the users in the environment, since a wall-mounted socket has a fixed location, and devices plugged into it via the insert will be within a limited distance of that location under normal circumstances.

In a further variant, at least one of the inserts analyses characteristics of current drawn by at least one of a number of appliances drawing current through the insert to identify those of the number of appliances drawing current.

This variant is able to cope with power strips connected to the wall-mounted socket via the insert, in that it can distinguish between current use by the individual appliances plugged in to the respective sockets of the power strip.

In an embodiment, the second data representative of at least one characteristic of electrical current is obtained from each of a number of monitoring devices in association with data identifying the monitoring devices from which they originated.

In this embodiment, users can be located, in that the data identifying the monitoring device can include data representative of the monitoring device's (relative) location or can be cross-referenced to a database associating each identifiable monitoring device with a (relative) location.

An embodiment of the method includes:
- providing a user interface for entering data relating to a monitoring device and
- storing entered data in association with an identification of the monitoring device to which it relates.

This embodiment can be used to locate users in that the data stored in association with an identification of the monitoring device can include data for locating the monitoring device. The embodiment can also be used to make the interpretation of the physiological signal or signals more accurate, because an indication of the types of appliances connected to the monitoring device can be included in the data stored in association with the identification of the monitoring device. Thus, for example, the monitoring device to which a television set is connected can be identified as such. The fact that it is drawing current informs the system executing the method of the fact that someone is watching television. The current levels may even enable the system to infer the volume at which the television is playing.

An embodiment of the method includes at least one of:
a) causing current supply to at least one appliance to be cut off when an absence in the environment of systems for obtaining the at least one physiological signal is detected; and
b) causing current supply to at least one appliance to be restored when a presence in the environment of at least one system for obtaining the at least one physiological signal is detected.

This embodiment uses the absence or presence of devices typically carried on the human body in a particular environment as a trigger for switching appliances on and off. An added energy-saving effect is thus obtained using the same components as are used to process physiological signals for the purpose of adapting outputs of devices in that environment.

According to another aspect, the signal processing system according to the invention includes:
- a portable device comprising:
   - an interface for obtaining at least one physiological signal from a system arranged to monitor at least one user, and
   - an interface for obtaining first data from a system for monitoring at least one characteristic of electrical current drawn by at least one appliance in an environment in which the at least one user is present, wherein the first data is received by the portable device through at least one wireless communications link, wherein the second data is obtained by means of at least one monitoring device for monitoring at least one characteristic of electrical current provided through a socket associated with the monitoring device,
   wherein the signal processing system is configured to determine a position of a user based on characteristics of wireless communication links between the portable device and each of a number of devices including at least a respective one of the monitoring devices,
- wherein the signal processing system is configured to use the first data to determine an output based on the at least one physiological signal and the position of the user in relation to at least one of the at least one appliance.

The system Can provide, or cause to be provided, an output more appropriate to the state of the user characterized by the physiological signal values.

In an embodiment, the system is configured to carry out a method according to the invention.

According to another aspect of the invention, there is provided a computer program including a set of instructions capable, when incorporated in a machine-readable medium, of causing a system having information processing capabilities to perform a method according to the invention.

### BRIEF DESCRIPTION OF THE DRAWINGS

The invention will be explained in further detail with reference to the accompanying drawings, in which:
Fig. 1 is a block diagram of an environment in which a body sensor network and current monitors are present;
Fig. 2 is a perspective view of an insert including a current monitor;
Fig. 3 is a flow chart of a method of initializing a device including a current monitor; and
Fig. 4 is a flow chart of a method of processing physiological signals.

### DETAILED DESCRIPTION

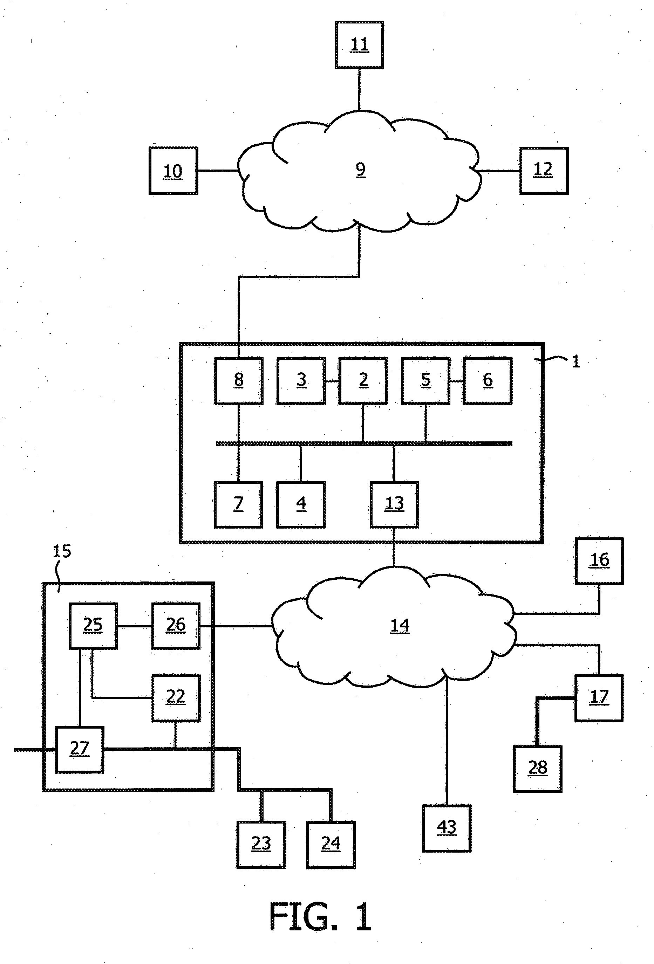

Fig. 1 illustrates an embodiment of a system for interpreting physiological signals, which is arranged to adjust its operation appropriately or causes the operation of an external device to be adjusted appropriately. In particular, the output of the system or external device can be adjusted in a manner appropriate to a particular mental state, especially the mood, of a user. For example, perceptible output of a content rendering device can be adjusted or caused to be adjusted, for example by selecting different content data or by adjusting settings of the device rendering the content data in perceptible form. Similarly, an ambient system can be adjusted at least partly on the basis of the mental state of at least one user as determined by interpreting physiological signals. The outputs of such an ambient system include, depending on the embodiment, at least one of ambient lighting parameters (color, intensity, direction, for example), air flow, temperature, humidity, haptic feedback and the like.

In the illustrated embodiment, a user is provided with a portable data processing device 1, e.g. a Personal Digital Assistant (PDA), smart phone or a dedicated device. The portable device 1 includes a central processing unit 2 and main memory 3, as well as a data storage device 4 (e.g. a solid state or magnetic non-volatile memory device). In the illustrated embodiment, it also includes a display driver 5 and a display 6, as well as a user input device 7, e.g. at least one of a keyboard, pointing device and touch-sensitive device combined with the display 6. Through these components, the portable device 1 is capable of providing a Graphical User Interface (GUI).

The portable data processing device 1 is generally battery-powered. It will generally be carried on the user's person. The portable data processing, device 1 also has a network interface 8 to a personal area network (PAN 9), e.g. implemented according to the IrDA, Bluetooth, UWB, Z-Wave or ZigBee standard.

Physiological sensors 10-12 are also connected to the PAN 9, through which they provide data representative of at least one respective physiological signal to the portable data processing device 1. Examples of physiological parameters that can be quantified by the signals include: heart rate, respiration rate, skin conductance, number of skin conductance responses, body temperature, heart rate variability, muscle activity, coherence between the respiration rate and heart rate sinus, etc. These are parameters that have been found to be connected to the mental state, in particular the mood, of a human being. It will be appreciated that the measurements by a physiological sensor 10-12 can be contactless or that the sensor 10-12 can be maintained in direct contact to the skin, depending on the physiological signal being provided. In one variant, one or more of the physiological sensors 10-12 is stationary, but is integrated into the PAN 9 when the portable data processing device 1 is within range. Thus, for example, a sensor 10-12 can be included in a chair, and establish contact with a user's portable data processing device 1 when the user sits down. In another variant, one or more physiological sensors are integrated into the portable data processing device 1, e.g. pressure sensors integrated into the user input device 7.

The portable data processing device 1 also comprises a second network interface 13, namely to a second wireless network 14, which can be a wireless local area network or a wide area network. The second network 14 can include a wireless cellular network or a wireless local area network in accordance with one of the IEEE 802.11x standards. The second network 14 enables the portable data processing device 1 to exchange data with wall-socket inserts 15-17, of which three are shown by way of example in Fig. 1. In an alternative embodiment, the wall-socket inserts 15-17 communicate by means of a power-line network (e.g. in accordance with the G.hn/G.9960 standard proposal) and a gateway between the second network 14 and such a power-line network.

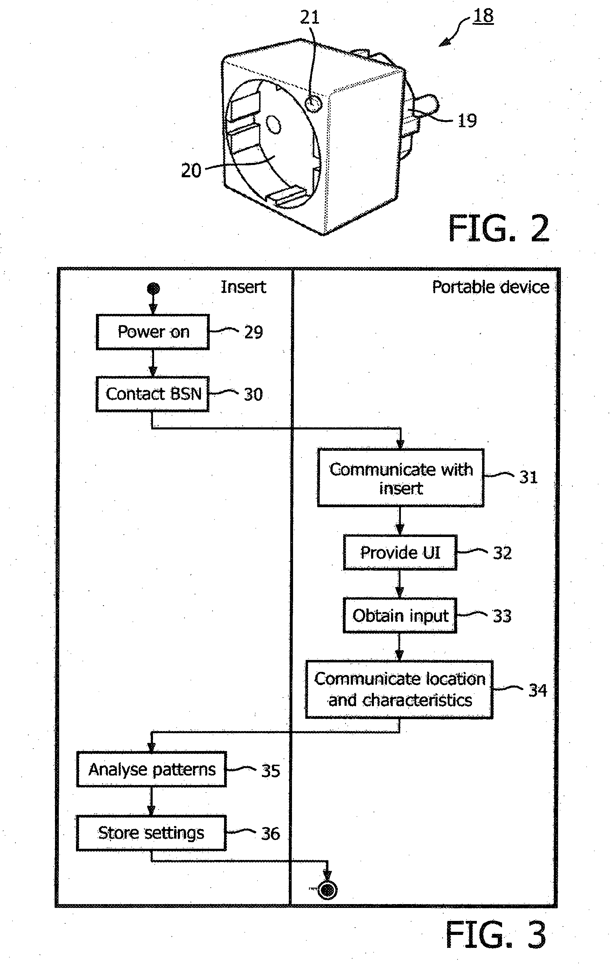

Fig. 2 shows a particular embodiment 18 of a wall-socket insert, which includes a plug 19 for insertion in a wall-mounted socket and a socket 20 replicating the wall-mounted socket, for receiving a power plug (not shown) of an electrical appliance. A button 21 for activating the wall-socket insert 18 is also provided.

By way of example, Fig. 1 illustrates that a first wall-socket insert 15 includes a monitoring device 22 for monitoring at least one characteristic of electrical current drawn by respective electrical appliances 23,24 by way of the first wall-socket insert 15. The first wall-socket insert 15 includes a processing device 25 for processing the measured values and providing data based on these measured values. The data is communicated to the portable data processing device 1 using a network interface 26 to the second network 14. It is noted that the first wall-socket insert 15 is used to obtain any one of a number of characteristics of the current drawn by the respective appliances 23,24, including voltage, current, powder, a moving average of the power consumption, frequency, phase angle between voltage and current, etc. The first wall-socket insert 15 can in particular also detect the presence of patterns in the voltage and/or current Indeed, such patterns can be used to distinguish between the two electrical appliances 23,24, for example to detect which one is operational or which characteristics (e.g. power consumption) are attributable to which one. The first wall-socket insert 15 of Fig. 1 also includes a switch 27 for switching the power supply to the electrical appliances 23,24 on and off.

The other wall-socket inserts 16,17 are similar to the first wall-socket insert 15. In the illustrated example, a third appliance 28 is connected to a second wall-socket insert 16, whilst no appliance is plugged into a third wall-socket insert 17.

In the illustrated embodiment, the wall-socket inserts 15-18 are initialized in the manner indicated in Fig. 3.

In a first step 29, the wall-socket insert 15-18 is plugged into a wall-mounted socket, or otherwise powered up (e.g. by pressing the button 21). The wall-socket insert 15-18 polls the wireless network 14 to discover the portable data processing device 1 (step 30).

The portable data processing device 1 responds (step 31) according to a predetermined protocol to set up a link to the wall-socket insert 15-18.

In the illustrated embodiment, it also provides (step 32) a Graphical User Interface (GUI) on the display 6, allowing a user to enter data relating to the wall-socket insert 15-18. This data comprises at least one of a name of the wall-socket insert, one or more names of the electrical appliances 23,24,28 or of the type thereof connected to the wall-socket insert 15-18, the name or the type of the room in which the wall-socket insert 15-18 is provided and the location (e.g. as a point on a compass).

Having obtained the user input (step 33), the portable data processing device 1 communicates the location and further relevant characteristics to the wall-socket insert 15-18 (step 34). In another embodiment, the portable data processing device 1 stores this data locally in a database.

The wall-socket insert 15-18 analyses patterns of power usage by connected electrical appliances 23,24,28 (step 35), learning how to distinguish between the different devices by, means of their electrical signature. After this first initialization, the wall-socket insert 15-18 stores its settings (step 36), whereupon it is ready to provide context information to the portable data processing device 1 with the connected physiological sensors 10-12. The context information provides valuable input for evaluating the psychophysiological signals measured by the body sensor network (BSN) comprising the physiological sensors 10-12.

One function that the wall-socket inserts 15-18 can also fulfill is an energy-saving function. Having been provided (step 34) with information on the types of electrical appliance 23,24,28 connected to it, the first wall-socket insert 15, for example, which is provided with a switch 27, can switch the current supply to the electrical appliances 23,24 on and off in dependence on whether a portable data processing device 1 with connected BSN is detected. To this end, the first wall-socket insert 15 can simply poll the network 14 at intervals to determine whether the device 1 is present. If neither it nor a similar device is present, then the current supply is switched off. Once the portable device 1 is detected again, the current supply is restored. The wall-socket insert 15 will fulfill this function only if informed that an appropriate electrical appliance 23,24 is arranged to draw current via it (e.g. a lighting appliance or the like).

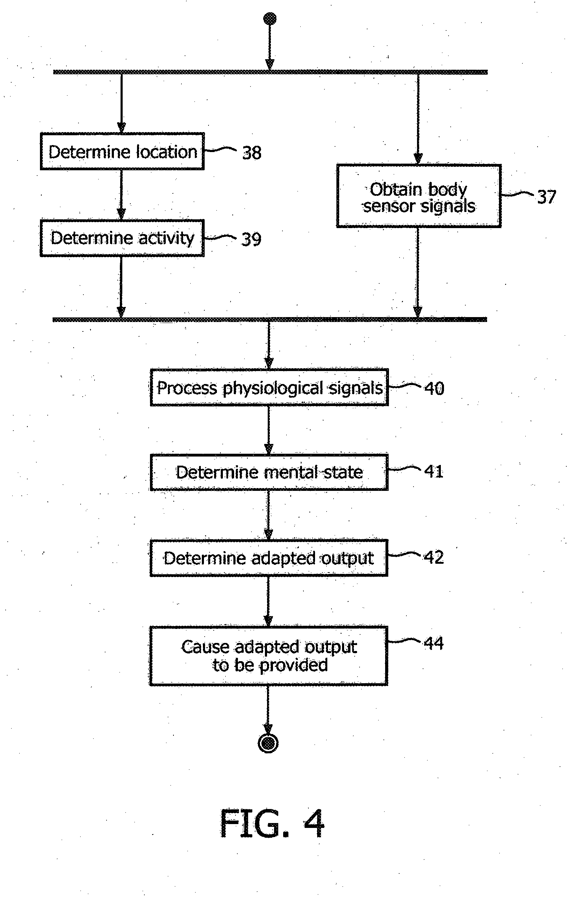

The process of evaluating the psychophysiological signals measured by the body sensor network (BSN) comprising the physiological sensors 10-12 is illustrated in outline in Fig. 4. The portable data processing device 1 obtains the physiological signals via the PAN 9 (step 37). It also determines the location of the user (step 38) by multilateration using the wireless signals received from the wall-socket inserts 15-18. Other methods of determining the user's location are conceivable, but a determination based on characteristics of wireless communication links between the portable data processing device 1 and the wall-socket inserts 15-18 requires no additional equipment.

The portable data processing device 1 also receives reports of activity from the wall-socket inserts 15-18 (step 39), which are based on data representative of at least one characteristic of electrical current drawn by any appliances 23,24,28 connected to the respective wall-socket insert 15-18. For example, one of the wall-socket inserts 15-18 can report that a coffee machine or a dish washer is being used, or has just been used.

The portable data processing device 1 processes the physiological signals (step 40) to determine the mental state, in particular the mood, of the user (step 41) and an appropriate response to the mental state in the circumstances (step 42). At least one of these steps 40-42 makes use of the location and activity information provided in the preceding steps 38,39, to adjust the algorithms that are based on data based on or corresponding to the physiological data.

As an example, if the user is determined to be in the vicinity of a television set 43 and the physiological signals indicate frustration, then the signals are interpreted as meaning that the user is watching a program he or she doesn't like. The appropriate response will be to determine an output that causes a switch to a different channel (step 44), and providing an appropriate command to the television set 43 over the second network 14 or another communications link. In another example, the portable data processing device 1 can determine that the user is near a coffee machine (step 38). The appropriate output would then be a command to brew a more soothing type of coffee.

In another example, the portable data processing device 1 can determine that the user is near a coffee machine (step 38) that has just been used (step 39). This information is then used to adapt the process of determining the user's mental state on the basis of the physiological signals (steps 40,41), depending on the type of physiological signal. It is known, for example, that a person's galvanic skin response reacts strongly to the intake of caffeine. Thus, in this use case, the first two steps 40,41 in the process of providing an appropriate output are adapted to the location and activity data.

In yet another embodiment, the portable data processing device 1 can adapt its own output in dependence on the physiological signals using the context information it has received from the wall-socket inserts 15-18, e.g. if it is provided with the resources to render audio tracks in perceptible form. Thus, the portable data processing device 1 can both provide and cause to be provided an output that the user is aware of and that is based on his or her inferred mental state.

It should be noted that the above-mentioned embodiments illustrate, rather than limit, the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The mere fact that certain measures are recited in mutually different dependent claims does not indicate that a combination of these measures cannot be used to advantage.

In an embodiment, a stationary device is used instead of the portable data processing device 1. The stationary device establishes a link to a body sensor network comprising at least one physiological sensor and a wireless transceiver when the body sensor network is in range.

In that embodiment, or in yet another embodiment, the GUI for providing data associated with current monitoring devices is provided by a stationary device, e.g. the television set 43, in combination with an appropriate remote control unit (not shown).

In another embodiment, a wall-socket insert corresponding to the wall-socket inserts 15-18 described above, but adapted for use with analogue telephones and comprising a socket and plug, e.g. according to the RJ11 or BS 6312 standard is used in addition to the wall-socket inserts 15-17, described.

## Claims

1. Method of signal processing, including:
- obtaining, by a portable data processing device (1), least one physiological signal from a system (10-12) arranged to monitor at least one user,
- obtaining, by the portable data processing device (1), first data, received by the portable data processing device (1) through at least one wireless communications link; the first data at least based on second data, obtained by means of at least one monitoring device (22) for monitoring at least one characteristic of electrical current provided through a socket associated at least temporarily with the monitoring device (22), the second data representative of at least one characteristic of electrical current drawn by at least one appliance (23,24,28) in an environment in which the at least one user is present,
- determining, by the portable data processing device (1), a position of a user based on characteristics of wireless communication links between the portable data processing device (1) and each of a number of devices (15-17;18) including at least a respective one of the monitoring devices (22), and
- determining, by the portable data processing device (1), using the first data, an output based on the at least one physiological signal and the position of the user in relation to the at least one appliance.

2. Method according to claim 1,
wherein each monitoring device (22) is comprised in an insert (15-17;18), the insert (18) including a plug (19) for insertion in a wall-mounted socket and at least one socket (20) for receiving a power plug of an electrical appliance (23,24,28).

3. Method according to claim 2,
wherein at least one of the inserts (15) analyses characteristics of current drawn by at least one of a number of appliances (23,24) drawing current through the insert (15) to identify those of the number of appliances (23,24) drawing current.

4. Method according to claim 1,
wherein the second data representative of at least one characteristic of electrical current is obtained from each of a number of monitoring devices (22) in association with data identifying the monitoring devices (22) from which they originated.

5. Method according to claim 1, including:
- providing a user interface for entering data relating to a monitoring device (22) and
- storing entered data in association with an identification of the monitoring device (22) to which it relates.

6. Method according to claim 1, including at least one of:
a) causing current supply to at least one appliance (23,24,28) to be cut off when an absence in the environment of systems (9-12) for obtaining the at least one physiological signal is detected; and
b) causing current supply to at least one appliance (23,24,28) to be restored when a presence in the environment of at least one system (9-12) for obtaining the at least one physiological signal is detected.

7. Computer program including a set of instructions capable, when incorporated in a machine-readable medium, of causing a system having information processing capabilities to perform a method according to any one of claims 1-6.

## Patentansprüche

1. Verfahren zur Signalverarbeitung, das Folgendes umfasst:
- seitens einer portablen Datenverarbeitungsvorrichtung (1) Erlangen von mindestens einem physiologischen Signal von einem System (12), das dafür ausgelegt ist, mindestens einen Benutzer zu überwachen,
- seitens einer portablen Datenverarbeitungsvorrichtung (1) Erlangen von ersten Daten, die durch die portable Datenverarbeitungsvorrichtung (1) über mindestens eine drahtlose Kommunikationsverbindung empfangen werden; wobei die ersten Daten zumindest auf zweiten Daten basieren, die mithilfe von mindestens einer Überwachungsvorrichtung (22) zum Überwachen von mindestens einer Eigenschaft des über eine Buchse, die zumindest vorübergehend der Überwachungsvorrichtung (22) zugeordnet ist, bereitgestellten elektrischen Stroms, wobei die zweiten Daten mindestens eine Eigenschaft des elektrischen Stroms darstellen, der durch mindestens ein Gerät (23, 24, 28) in einer Umgebung gezogen wird, in der sich der mindestens eine Benutzer befindet,
- seitens der portablen Datenverarbeitungsvorrichtung (1) Ermitteln einer Position eines Benutzers basierend auf Eigenschaften von drahtlosen Kommunikationsverbindungen zwischen der portablen Datenverarbeitungsvorrichtung (1) und jedem einer Anzahl an Vorrichtungen (15-17; 18) einschließlich mindestens einer betreffenden der Überwachungsvorrichtungen (22), und
- seitens der portablen Datenverarbeitungsvorrichtung (1) unter Verwendung der ersten Daten Ermitteln einer Ausgabe basierend auf dem mindestens einen physiologischen Signal und der Position des Benutzers in Bezug auf das mindestens eine Gerät.

2. Verfahren nach Anspruch 1
wobei jede Überwachungsvorrichtung (22) in einem Einsatz (15-17; 18) enthalten ist, wobei der Einsatz (18) einen Stecker (19) zum Einführen in eine Wandsteckdose und mindestens eine Buchse (20) zum Aufnehmen eines Stromsteckers eines elektrischen Geräts (23, 24, 28) umfasst.

3. Verfahren nach Anspruch 2,
wobei mindestens einer der Einsätze (15) Eigenschaften des Stroms analysiert, der durch mindestens eines von einer Anzahl an Geräten (23, 24) gezogen wird, die Strom durch den Einsatz (15) ziehen, um diejenigen Geräte der Anzahl an Geräten (23, 24) zu identifizieren, die Strom ziehen.

4. Verfahren nach Anspruch 1,
wobei die zweiten Daten, die mindestens eine Eigenschaft des elektrischen Stroms darstellen, von jeder einer Anzahl an Überwachungsvorrichtungen (22) in Verbindung mit Daten erlangt werden, die die Überwachungsvorrichtungen (22) identifizieren, von denen sie stammen.

5. Verfahren nach Anspruch 1 das Folgendes umfasst:
- Bereitstellen einer Benutzeroberfläche zum Eingeben von Daten in Bezug auf eine Überwachungsvorrichtung (22) und
- Speichern der eingegebenen Daten in Verbindung mit einer Identifikation der Überwachungsvorrichtung (22), auf die sie sich beziehen.

6. Verfahren nach Anspruch 1, das mindestens einen der folgenden Schritte umfasst:
a) Veranlassen, dass die Stromversorgung für mindestens ein Gerät (23, 24, 28) abgeschaltet wird, wenn eine Abwesenheit in der Umgebung der Systeme (9-12) zum Erlangen des mindestens einen physiologischen Signals detektiert wird; und
b) Veranlassen, dass die Stromversorgung für mindestens ein Gerät (23, 24, 28) wiederhergestellt wird, wenn eine Anwesenheit in der Umgebung von mindestens einem System (9-12) zum Erlangen des mindestens einen physiologischen Signals detektiert wird.

7. Computerprogramm mit einem Anweisungssatz, das, wenn es in ein maschinenlesbaren Medium aufgenommen ist, in der Lage ist, ein System mit Informationsverarbeitungsfähigkeiten zu veranlassen, ein Verfahren nach einem der Ansprüche 1 bis 6 durchzuführen.

## Revendications

1. Procédé de traitement de signal, comprenant :
- l'obtention, par un dispositif portatif de traitement de données (1), d'au moins un signal physiologique à partir d'un système (12) agencé pour surveiller au moins un utilisateur,
- l'obtention, par le dispositif portatif de traitement de données (1), de premières données, reçues par le dispositif portatif de traitement de données (1) par l'intermédiaire d'au moins une liaison de communication sans fil ; les premières données étant au moins fondées sur des secondes données, obtenues au moyen d'au moins un dispositif de surveillance (22) pour surveiller au moins une caractéristique d'un courant électrique fourni par l'intermédiaire d'une prise associée au moins temporairement au dispositif de surveillance (22), les secondes données étant représentatives d'au moins une caractéristique d'un courant électrique soutiré par au moins un appareil (23, 24, 28) dans un environnement dans lequel l'au moins un utilisateur est présent,
- la détermination, par le dispositif portatif de traitement de données (1), d'une position d'un utilisateur en fonction de caractéristiques de liaisons de communication sans fil entre le dispositif portatif de traitement de données (1) et chacun parmi un nombre de dispositifs (15-17 ; 18) comprenant au moins un respectif parmi les dispositifs de surveillance (22), et
- la détermination, par le dispositif portatif de traitement de données (1), en utilisant les premières données, d'une sortie en fonction de l'au moins un signal physiologique et de la position de l'utilisateur par rapport à l'au moins un appareil.

2. Procédé selon la revendication 1,
dans lequel chaque dispositif de surveillance (22) est compris dans une pièce rapportée (15-17 ; 18), la pièce rapportée (18) comprenant une fiche (19) pour l'insertion dans une prise montée sur mur et au moins une prise (20) pour recevoir une fiche électrique d'un appareil électrique (23, 24, 28).

3. Procédé selon la revendication 2,
dans lequel au moins une des pièces rapportées (15) analyse des caractéristiques d'un courant soutiré par au moins un parmi un nombre d'appareils (23, 24) soutirant un courant à travers la pièce rapportée (15) pour identifier ceux, parmi le nombre d'appareils (23, 24), soutirant du courant.

4. Procédé selon la revendication 1,
dans lequel les secondes données représentatives d'au moins une caractéristique de courant électrique sont obtenues à partir de chacun parmi un nombre de dispositifs de surveillance (22) en association avec des données identifiant les dispositifs de surveillance (22) à partir desquels elles proviennent.

5. Procédé selon la revendication 1, comprenant :
- la fourniture d'une interface utilisateur pour entrer des données concernant un dispositif de surveillance (22), et
- le stockage de données entrées en association avec une identification du dispositif de surveillance (22) auquel elles se rapportent.

6. Procédé selon la revendication 1, comprenant au moins un de :
a) l'entraînement de la coupure d'une alimentation en courant à au moins un appareil (23, 24, 28) lorsqu'une absence dans l'environnement de systèmes (9-12) pour obtenir l'au moins un signal physiologique est détectée ; et
b) l'entraînement de la restauration d'une alimentation en courant à au moins un appareil (23, 24, 28) lorsqu'une présence dans l'environnement d'au moins un système (9-12) pour obtenir l'au moins un signal physiologique est détectée.

7. Programme d'ordinateur comprenant un jeu d'instructions capable, lorsqu'il est incorporé dans un support lisible par machine, d'entraîner la réalisation, par un système possédant des capacités de traitement d'informations, d'un procédé selon une quelconque des revendications 1 à 6.
